(19) Europäisches Patentamt
European Patent Office
Office européen des brevets

(11) Veröffentlichungsnummer : **0 073 771**
**B1**

(12) # EUROPÄISCHE PATENTSCHRIFT

(45) Veröffentlichungstag der Patentschrift : **18.09.85**

(51) Int. Cl.⁴ : **C 07 C 49/417**, C 07 C 49/613, C 07 C 49/653, C 07 C 45/56, C 07 C 45/62, C 11 B 9/00, A 61 K 7/00

(21) Anmeldenummer : **82900537.0**

(22) Anmeldetag : **25.02.82**

(86) Internationale Anmeldenummer : **PCT/CH 82/00029**

(87) Internationale Veröffentlichungsnummer : **WO/8203216 (30.09.82 Gazette 82/23)**

(54) **NEUE RIECHSTOFFE.**

(30) Priorität : **13.03.81 CH 1718/81**
**11.02.82 CH 841/82**

(43) Veröffentlichungstag der Anmeldung : **16.03.83 Patentblatt 83/11**

(45) Bekanntmachung des Hinweises auf die Patenterteilung : **18.09.85 Patentblatt 85/38**

(84) Benannte Vertragsstaaten : **CH DE FR GB LI NL**

(56) Entgegenhaltungen :
**US-A- 3 578 686**
**US-A- 4 173 585**

(73) Patentinhaber : **L. GIVAUDAN & CIE Société Anonyme**
**CH-1214 Vernier-Genève (CH)**

(72) Erfinder : **BAUDIN, Josiane**
**6, rue René Naudin**
**F-74100 Annemasse (FR)**
Erfinder : **GONZENBACH, Hans Ulrich**
**61, rue de Lyon**
**CH-1203 Genf (CH)**

(74) Vertreter : **Urech, Peter, Dr. et al**
**Grenzacherstrasse 124 Postfach 3255**
**CH-4002 Basel (CH)**

**EP 0 073 771 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Riechstoffe. Es handelt sich dabei um die Verbindungen der Formel

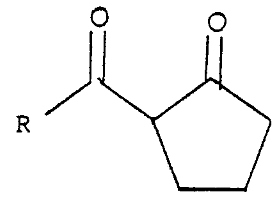

(I)

worin R einen aliphatischen Rest der Formel

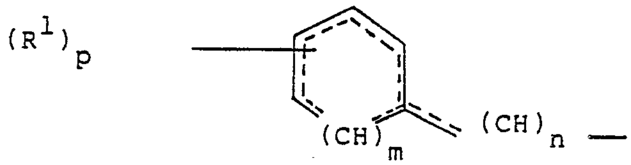

bedeutet, worin $R^1$ $C_{1-4}$-Alkyl, $C_{1-4}$-Alkyliden oder $C_{2-4}$-Alkenyl ist, wobei die Summe der C-Atome in diesen Resten 2-5 beträgt, m und n = 0 oder 1 sind, p = 1, 2 oder 3 ist, und die gestrichelten Linien eine oder zwei fakultative Doppelbindungen darstellen.

Die Verbindungen der Formel I sind, mit der Ausnahme von 2-[3,3,5-Trimethyl-cyclohexylacetyl]-cyclopentanon, neue Verbindungen.

Die Alkyl-, Alkenyl- bzw. Alkylidenreste $R^1$ können geradkettig oder verzweigt sein ; Beispiele für Alkylreste sind Methyl, Aethyl, Propyl, Isopropyl, n-Butyl, sec. Butyl, tert. Butyl.

Beispiele für Alkenyl sind Vinyl, Propenyl, iso-Propenyl, Butenyl.

Beispiele für Alkyliden sind Methylen, Aethyliden, Propyliden, Isopropyliden, Butyliden.

$R^1$ ist vorzugsweise Methyl. p ist vorzugsweise 3. Im Falle von p = 2 oder 3 können die Reste $R^1$ gleich oder verschieden sein. m ist vorzugsweise gleich n ; besonders bevorzugt ist m = n = 1.

R stellt vorzugsweise einen der folgenden Reste dar :

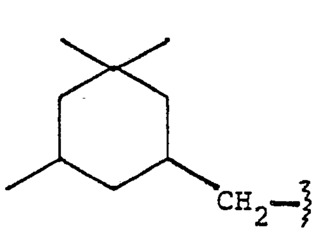
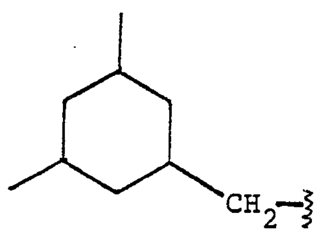
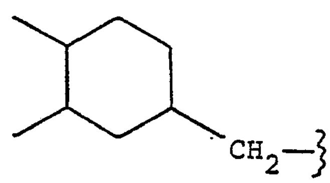

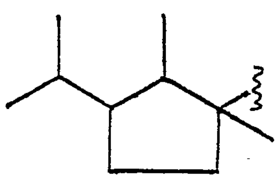
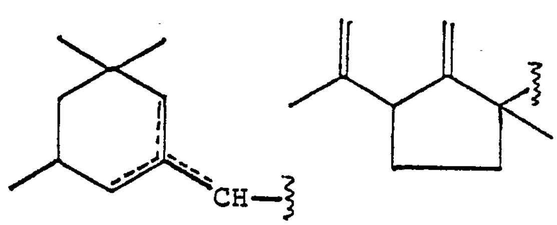

Verknüpfungsstelle mit dem Cyclopentanonrest

Der ersterwähnte Rest ist besonders bevorzugt.

Die Formel I soll auch die durch die Asymmetriezentren bedingten Stereoisomeren, die Doppelbindungsisomeren und die entsprechenden Enolformen der Diketone I umfassen. Die Enolformen der 1,3-Diketone sind :

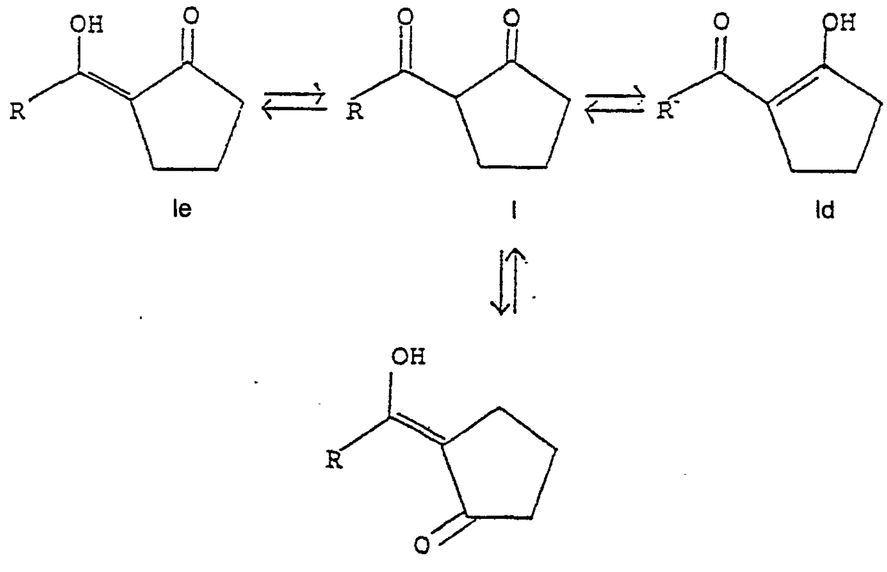

Ie I Id

Ic

Die Erfindung betrifft ferner ein Verfahren zur Herstellung der Verbindungen der Formel I.

Dieses Verfahren ist dadurch gekennzeichnet, dass man ein Enamin von Cyclopentanon mit einem Säurehalogenid der Formel

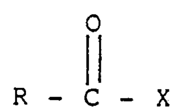

(II)

worin R obige Bedeutung hat und X Halogen darstellt, umsetzt, wobei die Herstellung von 2-[3,3,5-Trimethyl-cyclohexylacetyl]-cyclopentanon ausgeschlossen ist.

Als Säurehalogenide kommen insbesondere das Chlorid, Bromid und lodid in Frage, bevorzugt ist das Chlorid.

Die Herstellung der Diketone I kann demgemäss nach der bekannten Methodik der Enaminacylierung erfolgen, siehe beispielsweise G. Stork, A. Brizzolara, H. Landesmann, J. Szmuskovicz und R. Terrel in J. Amer. Chem. Soc. *85,* 207 (1963).

Als Enaminbildner können z. B. folgende sekundäre Amine verwendet werden :

Aliphatische sekundäre Amine, wie Dialkylamine, z. B. Diäthylamin, oder cyclische sekundäre Amine, wie z. B. Morpholin, Piperidin, Pyrrolidin. Bevorzugt ist Morpholin.

Die Umsetzung des Enamins mit dem Halogenid II wird zweckmässigerweise in einem Lösungsmittel und unter Ausschluss von Luft und Feuchtigkeit durchgeführt. Geeignete Lösungsmittel sind wasserfreie Lösungsmittel wie z. B. Methylenchlorid, Chloroform, Dioxan, Benzol, Toluol, Dimethylformamid, etc. ; bevorzugt sind Methylenchlorid und Chloroform.

Das molare Mengenverhältnis von Enamin zu Halogenid beträgt zweckmässigerweise ungefähr 1 : 1, doch kann das erstere auch im Ueberschuss z. B. in doppelter Menge, oder noch mehr, eingesetzt werden. Anstelle eines Ueberschusses an Enamin kann-zwecks Neutralisation der gebildeten Säure-Zugabe irgendeines Amins, z. B. Triäthylamin erfolgen.

Die Reaktionstemperatur liegt zweckmässigerweise zwischen 0 °C und der Rückflusstemperatur des Reaktionsgemisches, vorzugsweise bei etwa 40 °C. Bei dieser Temperatur belässt man das Gemisch beispielsweise mehrere Stunden. Hierauf kühlt man ab, zweckmässig auf Zimmertemperatur, und setzt alsdann eine Säure, z. B. Salzsäure zu. Die Aminabspaltung kann sodann durch nochmaliges Erwärmen, z. B. auf Rückflusstemperatur, erreicht werden.

Die Isolierung des Reaktionsproduktes kann nach an sich bekannten Methoden durchgeführt werden. Man nimmt z. B. das Reaktionsprodukt in einem organischen Lösungsmittel auf und wäscht die organische Lösung zuerst mit verdünnter Säure, wie einer 10 %igen HCl-Lösung. Nach Neutralwaschen und Trocknen kann das rohe Produkt durch übliche Methoden, wie z. B. Adsorptionschromatographie und/oder Destillation gereinigt werden.

Die Verbindungen I weisen besondere organoleptische Eigenschaften auf, auf Grund derer sie sich vorzüglich als Riechstoffe eignen.

Die Erfindung betrifft demgemäss auch die Verwendung der Verbindungen I als Riechstoffe.

Die Verbindungen I besitzen insbesondere holzig-animalische Geruchsnoten ; sie zeichnen sich insbesondere durch hohe Geruchsintensität verbunden mit ausserordentlichem Diffussionsvermögen und ungewöhnlicher Haftfestigkeit aus.

In geringen Konzentrationen eingesetzt, verstärken sie die Geruchsnoten von Riechstoffkompositionen, insbesondere von holzigen oder animalischen Kompositionen oder Basen, und verleihen diesen Wärme und Strahlungskraft.

Als Beispiele solcher Kompositionen seien Kompositionen mit Zedernholz-, Sandelholz- und Patchoulinoten sowie Costus- und Castoreum- (Bibergeil) Noten genannt.

Die Verbindungen eignen sich auch vorzüglich zur Modifizierung von Ledernoten und Chyprenoten, sowohl der femininen wie auch der masculinen Richtung.

Die Verbindungen I verbinden sich mit zahlreichen bekannten Riechstoffingredienzien natürlichen oder synthetischen Ursprungs, wobei die Palette der natürlichen Rohstoffe sowohl leicht- als auch mittel- und schwerflüchtige Komponenten, und diejenige der Synthetika Vertreter aus praktisch allen Stoffklassen umfassen kann, wie dies aus der folgenden Zusammenstellung ersichtlich ist.

— Naturprodukte, graues Ambra, Bergamottöl, Bibergeil und Substitute, acetyliertes Cedernholzöl (z. B. Vertofix® IFF bzw. Cedartone® Givaudan), Eichenmoss, Estragonöl, Fichtennadelöl, Galbanumöl, Geraniumöl, Jasmin Absolue und seine Substitute, Lavandinöl, Lavendelöl, Mandarinenöl, Orangenöl, Osmanthus Absolue und Substitute Patchouliöl, Petitgrainöl Paraguay, Sandelholzöl, Vetiveröl, Wermutöl, Ylang-Ylang-Oel,

— Alkohole, wie Borneol, Cedrol, Citronellol, Eugenol, Geraniol, cis-3-Hexenol, Linalool, 3-Methyl-5-(2',2',3'-trimethyl-cyclopent-3'-en-1'-yl)-pentan-2-ol (Sandalore® Givaudan), Phenylpropylalkohol, Vetivenol, Zimtalkohol,

— Aldehyde, wie p-tert. Butyl-α-methyl-dihydro-zimt-aldehyd (z. B. Lilial® Givaudan), Citral, Decanal, 3,5-Dimethyl-cyclohex-3-en-carboxaldehyd, Heliotropin, α-Hexylzimtaldehyd, Hydroxycitronellal, Methylnonylacetaldehyd, 4-[4-Methyl-3-pentenyl-]-cyclohex-3-en-1-aldehyd (z. B. Myraldène® Givaudan), Syringaaldehyd, Vanillin.

— Ketone, wie Acetylcedren, α-Jonon, Kampher, Menthon, p-Methylacetophenon, Methyljonone,

— Ester, wie 3-Aethyl-1,1-dimethyl-cyclohex-3-en-2-carbonsäureäthylester (Givescone® Givaudan), Benzylacetat, Benzylsalicylat, Bornylacetat, p-tert. Butylcyclohexylacetat, Cedrylacetat, cis-3-Hexenylacetat, Linalylacetat, 4[4-Methyl-3-pentenyl-]-cyclohex-3-en-1-yl-carbinylacetat (z. B. Myraldylacetat® Givaudan), Methyldihydrojasmonat, Styrallylacetat,

— Lactone, wie γ-Undecalacton, Cumarin,

— Verschiedene weitere, in der Parfümerie oft benützte Komponenten wie Acetaldehyd-propylphenyl-äthylacetal, Methyl-1-methyl-cyclododecyläther (z. B. Madrox® Givaudan), Moschus-Verbindungen (Ambrette-, Keton-moschus, 12-Oxa-hexadecanolid (z. B. Musk 174® Naarden), 1,1-Dimethyl-4-acetyl-6-tert. butylindan, Indol), Skatol.

Die Verbindungen der Formel I lassen sich in weiten Grenzen einsetzen, die beispielsweise von 0,01 % (Detergentien)-10 % (alkoholische Lösungen) in Kompositionen reichen können, ohne dass diese Werte jedoch Grenzwerte darstellen sollen da der erfahrene Parfümeur auch mit noch geringeren Konzentrationen oder sogar nur Spuren Effekte erzielen oder aber mit noch höheren Dosierungen neuartige Komplexe aufbauen kann. Diè bevorzugten Konzentrationen bewegen sich zwischen 0,5 und 5 %. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Desodorantien, Detergentien, Tabak, etc.).

Die Verbindungen I können demgemäss bei der Herstellung von Kompositionen und — wie obige Zusammenstellung zeigt — unter Verwendung einer breiten Palette bekannter Riechstoffe, verwendet werden. Bei der Herstellung solcher Kompositionen können die oben aufgeführten bekannten Riechstoffe nach (dem Parfümer bekannter) Art und Weise verwendet werden, wie z. B. aus W. A. Poucher, Perfumes, Cosmetics and Soaps *2*, 7. Auflage, Chapman und Hall, London, 1974 hervorgehend.

Aus US-PS 4, 173, 585 (IFF) sind Riechstoffe mit Cyclopentanstruktur bekanntgeworden. Im Gegensatz zu den vorliegenden Verbindungen sind jene Verbindungen monofunktionelle Verbindungen, die alle zwingend einen trimethylsubstituierten Cyclopentylrest aufweisen, der mit einem, eine funktionelle Gruppe tragenden Fünf- oder Sechsring verbunden ist, und zwar über eine ungesättigte aliphatische Brücke.

Aus US-PS 3,578,686 (Sterling Drug Inc.) ist das 2-[3,3,5-Trimethyl-cyclohexylacetyl]-cyclopentanon als Zwischenprodukt bekanntgeworden.

## Herstellung der Ausgangsmaterialien

Dihydroisophoron

In einen 3-Liter Autoklaven, versehen mit Heizung, Kühlung und Rührer, gibt man 2,2 Kg Isophoron und 5 g 5 % Palladium auf Kohle als Katalysator. Es wird nun ein Wasserstoffdruck von 8-10 atm erzeugt und langsam auf 30-35 °C erwärmt. Da die Hydrierung exotherm ist, wird durch Kühlung dafür gesorgt, dass eine Temperatur von 50 °C nicht überschritten wird. Nach Beendigung der Wasserstoffaufnahme (Temperatur sinkt) erfolgt Abfiltrieren des Katalysators. Man erhält so 2,2 Kg rohes Dihydroisophoron, welches gaschromatographisch einheitlich ist und direkt weiterverwendet wird.

$n_D^{20}$ : 1,445 5

Ausbeute : quantitativ

1-Hydroxy-3,3,5-trimethyl-cyclohexylessigsäure-äthylester

In einen 6-Liter Kolben, der mit Rührer, Thermometer, Tropftrichter und Rückflusskühler versehen ist, gibt man 300 ml absoluten Aether, 150 ml trockenes Benzol und 170 g Magnesiumspäne. Nun wird ein Gemisch von 1,100 Kg Dihydroisophoron und 960 g Chloressigsäure-äthylester, gelöst in 1,200 l Aether und 600 ml Benzol, eingetropft. (Springt die Reaktion während der Zugabe der ersten 200 ml und Erwärmen auf leichten Rückfluss nicht von selbst an, so werden einige Kriställchen Jod zugegeben). Der Reaktionsbeginn ist erkennbar an Trübung und Wärmetönung. Eine Zugabe der Reagenzien wird so fortgesetzt, dass das Reaktionsgemisch nur mässig siedet, sie ist innert 1 1/4 Stunden beendet. Danach wird noch 3 1/2 Stunden bei Rückflusstemperatur gehalten und nach Abkühlung auf 2,5 Kg Eis und 1,2 Liter konz. Salzsäure gegeben. Man trennt die organischen Phase ab und wäscht mit Wasser (enthaltend 1 % HCl conz.), mit Wasser und noch mehrmals mit 10 %iger Natriumcarbonatlösung. Nach Abdampfen des Lösungsmittels im Vakuum verbleiben 1,69 Kg Rohprodukt, welche destilliert werden. Die Hauptfraktion (1,250 Kg) wird nochmals mit 10 %iger Natriumcarbonablösung gewaschen und nochmals destilliert (100 °C/3 mm Hg). Man erhält so 1,046 Kg 1-Hydroxy-3,3,5-trimethyl-cyclohexyl-essigsäure-äthylester.

$n_D^{20}$ : 1,458 0
Ausbeute : 62 %

3,3,5-Trimethyl-1-oder -6-cyclohexenyl bzw. cyclohexylidenessigsäure-äthylester

In einen 2-Liter Kolben, der mit Rührer, Thermometer und Rückflusskühler mit Wasserabscheider versehen ist, erhitzt man unter starkem Rühren 1,040 Kg 1-Hydroxy-3,3,5-trimethyl-cyclohexyl-essigsäure-äthylester, 35 g $KHSO_4$ und 300 ml Toluol. Bei einer Innentemperatur von 140-150 °C werden innert 4 Stunden 78 ml Wasser abgeschieden. Man kühlt hierauf ab, wäscht mit 10 %iger Natriumcarbonatlösung und verdampft das Toluol im Vakuum. Man erhält 990 g Rohprodukt welche destilliert werden und 880 g eines Isomerengemisches (GC : 3 peaks) liefern.

$n_D^{20}$ : 1,456 0 – 1,464 0
Ausbeute : 92 %

3,5-Dimethyl-cyclohexanon

In einen Rundkolben, der mit Rührer, Thermometer, Tropftrichter und Rückflusskühler versehen ist, gibt man 128 g 3,5-Dimethyl-cyclohexanol und 500 ml Benzol. Unter Rühren und Kühlen wird nun langsam ein Gemisch von 119 g $Na_2Cr_2O_7$, 500 ml Wasser, 162 ml konz. $H_2SO_4$ und 50 ml Eisessig zugetropft. Dabei soll die Temperatur + 10 °C nicht übersteigen. Es wird bei dieser Temperatur noch 3 Stunden weiter gerührt und dann die organische Phase abgetrennt. Die wässrige Phase wird mit 130 ml Wasser verdünnt und mit Benzol extrahiert. Die vereinigten organischen Phasen werden neutral gewaschen und eingeengt, der Rückstand über eine Widmerkolonne destilliert ; er ergibt 102,7 g 3,5-Dimethyl-cyclohexanon.

Sdp : 55 °C/6 mmHg $n_D^{20}$ : 1,443 2
Ausbeute : 82 %

3,4 Dimethyl-cyclohexanon
Entsprechende Oxidation von 3,4-Dimethylhexanol liefert 3,4-Dimethyl-cyclohexanon.
Sdp : 66 °C/11 mmHg

3,5-Dimethyl-cyclohexyliden-essigsäureäthylester

In einen Rundkolben, der mit Rührer, Thermometer, Tropftrichter und Rückflusskühler versehen ist, gibt man 85 g trockenes Toluol. Unter Stickstoffatmosphäre werden nun 17,2 g Natrium in kleinen Portionen eingetragen und das Gemisch wird zum Sieden erhitzt. Nach Lösen des Natriums (nach etwa 3 Stunden) wird auf 20 °C abgekühlt und langsam (während ca. 1 1/2 Stunden), ein Gemisch von 219 g Aethyl-diäthyl-phosphono-acetat, 95 g 3,5-Dimethylcyclohexanon und 220 g trockenem Toluol zugetropft. Die Temperatur wird zwischen 25 und 30 °C gehalten. Bei dieser Temperatur wird noch während zwei Tagen weiter gerührt und hierauf auf 400 g Eis gegossen, mit Toluol extrahiert, neutral gewaschen und eingeengt. Der Rückstand wird über eine Widmerkolonne destilliert und liefert so 110 g 3,5-Dimethylcyclohexylidenessigsäureäthylester.

Sdp : 58 °C/0,04 mmHg $n_D^{20}$ : 1,472 0
Ausbeute : 75 %

3,4-Dimethyl-cyclohexyliden-essigsäureäthylester

Bei Anwendung obiger Wittig-Horner-Bedingungen auf 3,4-Dimethyl-cyclohexanon wird 3,4-Dimethyl-cyclohexyliden-essigsäureäthylester erhalten.

Sdp : 56 °C/0,08 mmHg $n_D^{20}$ : 1,476 9

2-sec.Butyl-cyclohexyliden-essigsäureäthylester

Durch analoge Umsetzung von 2-sec.Butyl-cyclohexanon erhält man 2-sec.Butyl-cyclohexyliden-essigsäureäthylester.
Sdp : 92 °C/0,3 mmHg $n_D^{20}$ : 1,483 9

3,3,5-Trimethyl-cyclohexylessigsäure-äthylester

In einen 3-Liter Autoklaven, versehen mit Heizung, Kühlung und Rührer, gibt man 875 g 3,3,5-Trimethyl-1- oder 6-cyclohexenyl bzw. cyclohexylidenessigsäure-äthylester, 5 g 5 %iges Palladium auf Kohle und 25 g Natriumcarbonat. Es wird ein Wasserstoffdruck von 20 atm erzeugt und auf 75 °C erwärmt. Die Hydrierung verläuft schnell und exotherm und es muss deshalb gekühlt werden, damit die Temperatur von 100 °C nicht überschritten wird. Nach dem Erkalten wird filtriert. Man erhält in quantitativer Ausbeute 875 g eines Diastereomerengemisches (GC : zwei Peaks) von 3,3,5-Trimethyl-cyclohexylessigsäure-äthylester, welche direkt in der folgenden Stufe eingesetzt werden.

3,5-Dimethyl-cyclohexyl-essigsäureäthylester

In einen 1-Liter Autoklaven, versehen mit Heizung, Kühlung und Rührer, gibt man 54 g 3,5-Dimethyl-cyclohexyliden-essigsäureäthylester, 200 ml abs. Methanol und 3 g Raney-Nickel, welche 3 mal mit abs. Methanol gewaschen wurden. Unter einem Wasserstoffdruck von 40 atm. wird bei 100 °C hydriert. Nach 8 Stunden hört die Wasserstoffaufnahme auf. Es wird vom Katalysator abfiltriert, eingeengt und destilliert. Man erhält 52,3 g 3,5-Dimethyl-cyclohexyl-essigsäureäthylester.
Sdp : 56 °C/0,7 mmHg $n_D^{20}$ : 1,444 0
Ausbeute : 98 %

3,4-Dimethyl-cyclohexyl-essigsäureäthylester

Die analoge Hydrierung von 3,4-Dimethyl-cyclohexyliden-essigsäureäthylester liefert 3,4-Dimethyl-cyclohexyl-essigsäureäthylester.
Sdp : 103 °C/7 mmHg $n_D^{20}$ : 1,448 2

2-sec.Butyl-cyclohexyl-essigsäureäthylester

Die analoge Hydrierung von 2-sec.Butyl-cyclohexyliden-essigsäureäthylester ergibt 2-sec.Butyl-cyclohexyl-essigsäureäthylester.
Sdp : 88 °/0,4 mmHg $n_D^{20}$ : 1,459 0

3,3,5-Trimethyl-cyclohexylessigsäure

875 g 3,3,5-Trimethyl-cyclohexylessigsäure-äthyl-ester werden in 2,2 Liter Wasser mit 670 g 30 %iger Natronlauge versetzt und während 2 1/2 Stunden unter Rühren bei Rückflusstemperatur gehalten. Nach dem Abkühlen wird neutralisiert, mit 420 g $H_2SO_4$ 63 % angesäuert und mit Cyclohexan extrahiert. Es wird neutral gewaschen, eingedampft und die 770 g Rohrprodukt werden destilliert. Man erhält 630 g 3,3,5-Trimethyl-cyclohexylessigsäure. (Ausbeute : 82 %)
$n_D^{20}$ : 1,462 2

3,3,5-Trimethyl-1- oder 6-cyclohexenyl bzw. cyclohexylidenessigsäure

Durch analoge Verseifung von 3,3,5-Trimethyl-1-oder 6-cyclohexenyl bzw. cyclohexylidenessigsäure-äthylester erhält man in einer Ausbeute von 90 % 3,3,5-Trimethyl-1-oder 6-cyclohexenyl bzw. cyclohexylidenessigsäure.
Smp. 80-82 °C.

3,5-Dimethyl-cyclohexyl-essigsäure

44,7 mg 3,5-Dimethyl-cyclohexyl-essigsäureäthylester werden in 300 ml Methanol und 15 g KOH, gelöst in 140 ml Wasser versetzt, und durch 3-stündiges Erhitzen auf Rückflusstemperatur verseift. Die Aufarbeitung liefert 35,6 g eines festen Rohproduktes, welches direkt weiterverwendet wird. Eine Probe wird zwecks Analyse destilliert.
Sdp : 120 °C/7 mmHg
Rohausbeute : 93 %

3,4-Dimethyl-cyclohexyl-essigsäure

Die entsprechende Verseifung von 3,4-Dimethyl-cyclohexyl-essigsäureäthylester liefert 3,4-Dimethyl-cyclohexyl-essigsäure als flüssiges Rohprodukt.
Sdp : 152 °C/7 mmHg

2-sec.Butyl-cyclohexyl-essigsäure

Die analoge Verseifung von 2-sec.Butyl-cyclohexyl-essigsäureäthylester liefert 2-sec.Butyl-cyclohexyl-essigsäure als flüssiges Rohprodukt.
Sdp : 160 °C/0,3 mmHg

1-Methyl-2-methylen-3-(prop-1-en-2-yl)-cyclopentan-1-carbonsäure

In einen Rundkolben, der mit Rührer, Thermometer, Tropftrichter und Rückflusskühler versehen ist, gibt man 75 ml getrocknetes Tetrahydrofuran und 23 g Magnesiumspäne. Ohne Rühren gibt man langsam (innert 4 Stunden) eine Lösung von 112,4 g 1-Methyl-2-chlormethyl-3-(prop-1-en-2-yl)-cylopent-1-en in 100 ml Tetrahydrofuran zu. Man bringt das Reaktionsgemisch eine Stunde auf 60-65° und kühlt auf − 10° ab. Durch die auf − 10 °C abgekühlte Lösung wird eine Kohlendioxydstrom eingeleitet. Die Menge des eingeleiteten $CO_2$ wird gewogen ; sie beträgt 29,5 g. Das Reaktionsgemisch lässt man über Nacht bei Raumtemperatur stehen. Man kühlt auf 10 °C und zersetzt mit 300 ml einer 10 %igen HCl-Lösung. Die Säure Lösung wird mit Aether extrahiert, die gebildete organische Säure wird als Natriumsalz (Behandlung mit 10 %iger Natronlauge) aus dem Aether in die wässrige Phase aufgenommen, welche mittels Aether gewaschen wird. Nach Ansäuern mit 10 %iger ortho-Phosphorsäure wird mit Aether extrahiert und die ätherische Lösung mit Natriumsulfat getrocknet. Man verdunstet das Lösungsmittel und erhält 78,2 g (Ausbeute : 66 %) rohe 3-Isopropenyl-2-methylen-1-methyl-cyclopentan-carbonsäure, welche mit Hexan umkristallisiert werden.
Smp : 66,5-68 °C.

3,3,5-Trimethyl-cyclohexyl-acetylchlorid

In einen Rundkolben, der mit Thermometer, Rührer, Rückflusskühler und Tropftrichter ausgerüstet ist, werden 114 g 3,3,5-Trimethyl-cyclohexylessigsäure in 350 ml trokkenem Aether rasch mit 32,8 g Phosphortrichlorid versetzt und anschliessend noch 4 Stunden bei Rückflusstemperatur gehalten. Man dekantiert von der phosphorigen Säure ab und dampft den Aether im Vakuum ab. Es bleiben 128 g (Ausbeute : quantitativ) rohes 3,3,5-Trimethyl-cyclohexyl-acethylchlorid zurück.

3,3,5-Trimethyl-1- oder 6-cyclohexenyl bzw. cyclohexyliden-acetylchlorid

Dieses Säurechlorid wird durch analoge Behandlung von 3,3,5-Trimethyl-1- oder 6-cyclohexenyl bzw. cyclohexyliden-essigsäure erhalten. Ausbeute nach Destillation : 52 %.
$n_D^{20}$ : 1,475 0.

1-Methyl-2-methylen-3-(prop-1-en-2-yl)-cyclopentan-1-carbonsäurechlorid

Wird durch analoge Behandlung von 1-Methyl-2-methyl-en-3-(prop-1-en-2-yl)-cyclopentan-1-carbonsäure erhalten. Ausbeute nach Destillation : 51 %.
$n_D^{20}$ : 1,493 0.

3,5-Dimethyl-cyclohexyl-acetylchlorid

Dieses Ausgangsmaterial wird durch analoge Behandlung von 3,5-Dimethyl-cyclohexyl-essigsäure erhalten.
Sdp : 73 °C/1,5 mmHg $n_D^{20}$ : 1,460 3

3,4-Dimethyl-cyclohexyl-acetylchlorid

Dieses Ausgangsmaterial wird durch analoge Behandlung von 3,4-Dimethyl-cyclohexyl-essigsäure erhalten.
Sdp : 68 °C/0,35 mmHg $n_D^{20}$ : 1,469 2

2-sec.Butyl-cyclohexyl-acetylchlorid

Dieses Ausgangsmaterial wird durch analoge Behandlung von 2-sec. Butyl-cyclohexyl-essigsäure erhalten.
Sdp : 71 °C/0,12 mmHg $n_D^{20}$ : 1,480 0

« Referenzbeispiel »

2-[3,3,5-Trimethyl-cyclohexylacetyl]-cyclopentanon

In einen Rundkolben, versehen mit Thermometer, Rührer, Rückflusskühler und Tropftrichter gibt man 111 g frisch destilliertes Morpholinocyclopenten, 71,7 g Triäthylamin und 900 ml Chloroform und kühlt auf 0 °C ab. Unter Stickstoffatmosphäre werden 128 g rohes 3,3,5-Trimethyl-cyclohexyl-acetylchlorid in 200 ml Chloroform langsam zugetropft. Man rührt solange, bis der Kolbeninhalt eine Temperatur von 20 °C erreicht hat und erwärmt noch 7 Stunden auf 40 °C. Nach 12 Stunden werden 76,2 konz. Salzsäure in 190 ml Wasser zugegeben und der Kolbeninhalt wird während 2 1/2 Stunden bei Rückflusstemperatur gehalten. Danach wird die organische Phase abgetrennt, die wässrige Phase zweimal mit Chloroform ausgeschüttet und die vereinigten Chloroformextrakte wie folgt gewaschen: 3 mal mit 10 %iger Salzsäure, 1 × mit Wasser, 1 × mit Bicarbonatlösung und schliesslich noch mit Kochsalzlösung bis zum Neutralpunkt. Nun wird im Rotationsverdampfer eingeengt und das Rohprodukt (212 g) im Vakuum destilliert. Man erhält 90 g (Ausbeute : 58 %) 2-[3,3,5-Trimethyl-cyclohexylacetyl]-cyclopentanon.

Sdp. : 111,5 °C/0,25 mmHg

Geruch : intensiv holzig, animalisch ; äusserst diffusiv und gut haftend.

Beispiel

2-[3,3,5-Trimethyl-1 oder 6-cyclohexenyl bzw. cyclohexyliden-acetyl] cyclopentanon

Werden obige Bedingungen auf destilliertes 3,3,5-Trimethyl-1- oder 6-cyclohexenyl bzw. cyclohexyliden-acetylchlorid angewendet, so erhält man 2-[3,3,5-Trimethyl-1- oder 6-cyclohexenyl bzw. cyclohexyliden-acetyl] cyclopentanon in einer Ausbeute von 62 %.

Sdp : 103 °C/0,08 mmHg

Geruch ; holzig, animalisch ; sehr diffusiv.

2-[1-Methyl-2-methylen-3-(prop-1-en-2-yl)-cyclopentyl-carbonyl]-cyclopentanon.

Dieselbe Behandlung von destilliertem 1-Methyl-2-methylen-3-(prop-1-en-2-yl)-cyclopentan-1-carbonsäurechlorid liefert in 36,5 %iger Ausbeute 2-[1-Methyl-2-methylen-3-(prop-1-en-2-yl)-cyclopentyl-carbonyl]-cyclopentanon.

Sdp : 119 °C/0,58 mmHg

Geruch : stark holzig, zedrig.

2-[3,5-Dimethyl-cyclohexylacetyl]-cyclopentanon

Wird 3,5-Dimethyl-cyclohexyl-acetylchlorid obiger Behandlung unterworfen, so erhält man 2-[3,5-Dimethyl-cyclohexylacetyl]-cyclopentanon.

Sdp : 129 °C/0,1 mmHg $n_D^{20}$ : 1,495 0

Geruch : intensiv, holzig und animalisch, diffusiv und haftfest.

2-[3,4-Dimethyl-cyclohexylacetyl]-cyclopentanon

Obiges Verfahren liefert aus 3,4-Dimethyl-cyclohexylacetylchlorid das 2-[3,4-Dimethyl-cyclohexyl-acetyl]-cyclopentanon.

Sdp : 133 °C/0,8 mmHg $n_D^{20}$ : 1,508 0

Geruch : intensiv holzig, animalisch mit Minzenote, diffusiv und haftfest.

2-[2-sec.Butyl-cyclohexylacetyl]-cyclopentanon

Durch obige Behandlung von 2-sec.Butyl-cyclohexyl-acetylchlorid erhält man 2-[2-sec.Butyl-cyclohexyl-acetyl]-cyclopentanon.

Sdp : 137 °C/0,28 mmHg $n_D^{20}$ : 1,511 7

Geruch : animalisch, Zibet.

2-[1,2-Dimethyl-3-isopropyl-cyclopentyl-carbonyl]-cyclopentanon

9,6 g 2-[1-Methyl-2-methylen-3-(prop-1-en-2-yl)-cyclopentyl-carbonyl]-cyclopentanon werden in 100 ml abs. Alkohol mit 1 g 5 %iger Palladiumkohle versetzt und bei Normaldruck hydriert. Nach beendeter Wasserstoffaufnahme (8 Stunden) wird filtriert, eingeengt und an der hundertfachen Menge Kieselgel mit Toluol/Essigester = 1 : 2 chromatographiert. Es werden 1,7 g 2-[1,2-Dimethyl-3-isopropyl-cyclopentyl-carbonyl]-cyclopentanon erhalten, welche mit wenig (< 5 %) 2-[1,2-Dimethyl-3-isopropyl-cyclopent-2-en-1-yl-carbonyl]-cyclopentanon bzw. 2-[1,2-Dimethyl-3-isopropyliden-cyclopentyl-carbonyl]-cyclopentanon verunreinigt sind.

$n_D^{20}$ : 1,504 2

Geruch : intensiv holzig, diffusiv und haftfest.

In den folgenden Beispielen bedeuten :
« Verbindung Ia » « 2-[3,3,5-Trimethyl-cyclohexyl-acetyl]-cyclopentanon »,
« Verbindung Ib » « 2-[3,5-Dimethyl-cyclohexyl-acetyl]-cyclopentanon »,
« Verbindung Ic » « 2-[3,4-Dimethyl-cyclohexyl-acetyl]-cyclopentanon ».

Beispiel 2

a) Parfumerie-Base Richtung Fougère

| | Gewichtsteile |
|---|---|
| Lavandinöl | 140 |
| Geraniumöl afrikan. | 140 |
| Coumarin | 140 |
| Benzoe resinoid Siam | 140 |
| Baum-Moos absolut aloresine | 60 |
| Patchouliöl | 60 |
| Bergamott-öl | 60 |
| Vetiveröl Bourbon | 40 |
| Keton-Moschus | 60 |
| Xylol-Moschus | 60 |
| | 900 |
| Verbindung Ia | 100 |
| | 1 000 |

Durch Zusatz von 10 % der Verbindung Ia wird die in der Fougèrebase vorherrschende Coumarinnote zurückgedrängt. Gleichzeitig wird die Komposition leichter und frischer. Die Lavendelnoten werden in angenehmer Weise hervorgehoben.

b) Würzige Base

| | Gewichtsteile |
|---|---|
| Benzylacetat | 100 |
| Hydroxycitronellal | 100 |
| Phenyläthylalkohol | 100 |
| Amylsalicylat | 100 |
| Patchouliöl | 80 |
| Ylangöl | 50 |
| Eugenol | 50 |
| Linalylacetat | 60 |
| Keton-Moschus | 50 |
| Cedrylacetat | 30 |
| Epoxycedren | 30 |
| Vertofix cœur® (Acetylcedren) | 30 |
| Coumarin | 30 |
| Krauseminzöl | 15 |
| Thymianöl | 15 |
| Methylsalicylat | 5 |
| Zitronenöl | 5 |
| Dipropylenglykol (DPG) | 100 |
| | 950 |
| Ia | 50 |
| | 1 000 |

Der Zusatz von 5 % Ia bewirkt nicht nur eine Verstärkung der Geruchsintensität, sondern auch eine Verbesserung der Geruchsqualität der würzigen Base. Der Geruchskomplex erhält eindeutig mehr Volumen.

c) Parfumerie-Base Richtung Holz

| | Gewichtsteile |
|---|---|
| Madrox® Givaudan (1-Methyl-1-methoxy-cyclododecan) | 150 |
| Vetivenylacetat | 150 |
| Sandela® Givaudan (3-Isocamphyl-(5)-cyclohexanol) | 150 |

| | |
|---|---|
| Linalool | 100 |
| Patchouliöl | 50 |
| Ironal (6-Methyl-α-Ionon) | 50 |
| Linalylacetat | 50 |
| Citronellol | 50 |
| Benzylacetat | 30 |
| Baum-Moos farblos absolut | 30 |
| α-Amylzimtaldehyd | 20 |
| Methylnonylacetaldehyd 10 % in DPG | 20 |
| Eugenol | 20 |
| C-11-Aldehyd (10 % DPG) | 10 |
| Labdanumöl (frz.) | 10 |
| Sandalore® Givaudan (3-Methyl-5-(2,2,3-)trimethylcyclopent-3-en-1-yl) pentan-2-ol | 10 |
| | 900 |
| Ia | 100 |
| | 1 000 |

Durch Zusatz von 10 % Ia wird die Geruchsintensität der Holzbase in bedeutender Weise gesteigert. Die Patchoulinoten werden in vorteilhafter Weise in Richtung Zedern-, Leder- und animalische Noten modifiziert.

d) Parfumerie-Base Richtung Chypre

| | Gewichtsteile |
|---|---|
| α-Ionon | 200 |
| Ambrette-Moschus | 100 |
| Phenyläthylalkohol | 30 |
| Bergamottöl | 70 |
| Vertofix cœur | 50 |
| Jasmin synth. | 40 |
| Patchouliöl | 40 |
| Rhodinol | 30 |
| Nelkenbase | 30 |
| α-Hexylzimtaldehyd | 30 |
| Zibet-öl 10 % DPG | 20 |
| Sandalore® Givaudan | 10 |
| Armoise-essenz (Beifussöl) | 10 |
| C-11-Aldehyd 10 % DPG | 10 |
| Baum-Moos absolue | 50 |
| Styrallylacetat | 20 |
| DPG | 200 |
| | 990 |
| Ia | 10 |
| | 1 000 |

Im Falle dieser Chyprebase bewirkt bereits ein Zusatz von nur 1 % Ia eine Geruchsintensivierung. Gleichzeitig werden die Holznoten hervorgehoben und es wird der Zederncharakter betont.

e) Animalische Base

| | Gewichtsteile |
|---|---|
| Sandela® Givaudan | 100 |
| Madrox® Givaudan | 100 |
| Vertofix coeur® | 100 |
| Patchouliöl | 50 |
| Benzylsalicylat | 40 |
| Linalylacetat | 40 |
| Myrrhenöl | 30 |
| Benzoe resinoid Siam | 30 |
| Aethylenbrassylat | 30 |
| Castoreum synth. | 30 |
| C-11-Aldehyd 10 % DPG | 20 |
| C-12-Aldehyd L 10 % DPG | 20 |
| β-Ionon | 20 |
| p-Cresyl-phenylacetat | 5 |

| | |
|---|---|
| Indol | 5 |
| DPG | 330 |
| | 950 |
| Ia | 50 |
| | 1 000 |

Der Zusatz von 5 % Ia verleiht dieser Komposition eindeutig mehr Charakter und akzentuiert die Castoreum- und Ledernoten.

f) Würzige Parfumerie-Base

| | Gewichtsteile |
|---|---|
| Bergamott-öl | 200 |
| Patchouliöl | 200 |
| Sandelholzöl | 200 |
| Myrascone® Givaudan | 100 |
| Methyldihydrojasmonat | 70 |
| α-Ionon | 50 |
| p-tert.-Butyl-cyclohexanolacetat | 50 |
| Basilikumöl | 30 |
| | 900 |
| Ia | 100 |
| | 1 000 |

Durch Zusatz von 10 % Ia wirkt dieser Geruchskomplex aromatischer, würziger. Es erhält mehr « Volumen » und wirkt gleichzeitig harmonischer, abgerundeter.

g) Parfumerie-Base Richtung Chypre

| | Gewichtsteile. |
|---|---|
| Hydroxycitronellal | 100 |
| Bergamotte-öl | 80 |
| Methyldihydrojasmonat | 80 |
| α-Hexylzimtaldehyd | 80 |
| Phenyläthylalkohol | 80 |
| Baummoos absolut farblos | 40 |
| Patchouliöl | 40 |
| Linalool | 40 |
| α-Jonon | 40 |
| Keton-Moschus | 40 |
| Vetiveröl | 20 |
| Sandelholzöl | 20 |
| Benzylacetat | 20 |
| Styrallylacetat | 5 |
| Undecalacton | 5 |
| C-11-Aldehyd 10 % in DPG | 5 |
| Zibetöl 10 % in DPG | 5 |
| Dipropylenglykol | 200 |
| | 900 |
| Ib | 100 |
| | 1 000 |

Durch Zusatz von 10 % der Verbindung Ib erhält die Base eine holzig-zedrige Note, wie diese üblicherweise nur durch Zusatz von Zedernholzöl erzielt werden kann. Gleichzeitig wirkt die Base viel kräftiger, herber, frischer und auch trockener. Aus der eher femininen Grundkomposition ist eine Base geworden, die sich gut für ein Herren-Cologne eignet.

h) Parfumerie-Base in Richtung Tee

| | Gewichtsteile |
|---|---|
| Bergamotte-öl | 150 |
| Linalool | 100 |
| Hydroxycitronellal | 100 |
| Methyldihydrojasmonat | 60 |
| Patchouliöl | 40 |

| | |
|---|---|
| Basilikumöl | 30 |
| Methyleugenol | 20 |
| β-Jonon | 20 |
| Formiate oxyoctaline[R] (3,4,5,6,7,8,9,10-Octahydro-1,2-8,10-tetramethyl-5-formoxynaphthalin) | 10 |
| Galaxolide[R] (1,3,4,6,7,8-Hexahydro-4,6,6,7,8,8-hexamethyl-cyclopenta-γ-2-benzopyran) | 10 |
| Bornylacetat | 10 |
| 2,2,8-Trimethyl-7-nonen-3-ol | 10 |
| Baummoos absolut farblos | 10 |
| Zitronenöl | 10 |
| Vertofix[R] | 10 |
| Indol 10 % in Dipropylenglykol | 10 |
| Dipropylenglykol | 350 |
| | 950 |
| Ic | 50 |
| | 1 000 |

Durch Zusatz von 5 % der Verbindung Ic wird die ursprüngliche Base viel weicher und pudriger. Sie wirkt nun zudem blumig und angenehm herb, krautig und würzig.

**Patentansprüche**

1. Verbindungen der Formel

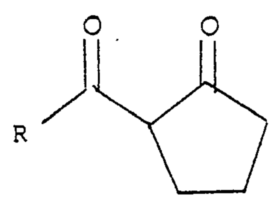

worin R einen aliphatischen Rest der Formel

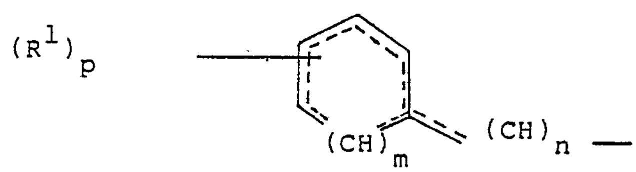

bedeutet, worin $R^1$ $C_1$-$C_4$-Alkyl, $C_{1\text{-}4}$-Alkyliden oder $C_{2\text{-}4}$-Alkenyl ist, wobei die Summe der C-Atome in diesen Resten 2-5 beträgt, m und n = 0 oder 1 sind, p = 1, 2 oder 3 ist, und die gestrichelten Linien eine oder zwei fakultative Doppelbindungen darstellen, mit der Ausnahme von 2-[3,3,5-Trimethyl-cyclohexylacetyl]-cyclopentanon.

2. 2-[3,3,5-Trimethyl-1-oder 6-cyclohexenyl bzw. cyclohexyliden-acetyl] cyclopentanon.
3. 2-[1-Methyl-2-methylen-3-(prop-1-en-2-yl)-cyclopentylcarbonyl]-cyclopentanon.
4. 2-[3,5-Dimethyl-cyclohexylacetyl]-cyclopentanon.
5. 2-[3,4-Dimethyl-cyclohexylacetyl]-cyclopentanon.
6. 2-[1,2-Dimethyl-3-isopropyl-cyclopentylcarbonyl] cyclopentanon.
7. 2-[2-sec.Butyl-cyclohexylactyl]-cyclopentanon.
8. Riechstoffkompositionen, gekennzeichnet durch einen Gehalt an einer Verbindung der Formel

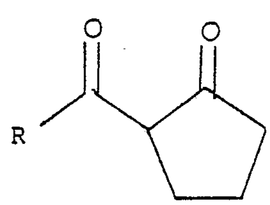

worin R einen aliphatischen Rest der Formel

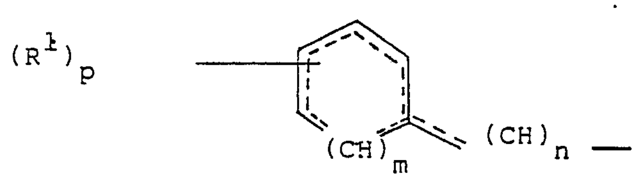

bedeutet, worin $R^1$ $C_{1-4}$-Alkyl, $C_{1-4}$-Alkyliden oder $C_{2-4}$-Alkenyl ist, wobei die Summe der C-Atome in diesen Resten 2-5 beträgt, m und n = 0 oder 1 sind, p = 1, 2 oder 3 ist, und die gestrichelten Linien eine oder zwei fakultative Doppelbindungen darstellen.

9. Riechstoffkomposition, gekennzeichnet durch einen Gehalt an 2-[3,3,5-Trimethyl-cyclohexylacetyl]-cyclopentanon.

10. Riechstoffkomposition, gekennzeichnet durch einen Gehalt an 2-[3,3,5-Trimethyl-1-oder 6-cyclohexenyl bzw. cyclohexyliden-acetyl] cyclopentanon.

11. Riechstoffkomposition, gekennzeichnet durch einen Gehalt an 2-[1-Methyl-2-methylen-3-(prop-1-en-2-yl)-cyclopentylcarbonyl]-cyclopentanon.

12. Verfahren zur Herstellung von Verbindungen der Formel

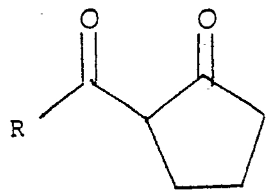

(I)

worin R einen aliphatischen Rest der Formel

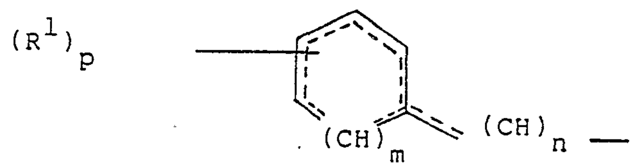

bedeutet, worin $R^1$ $C_{1-4}$-Alkyl, $C_{1-4}$-Alkyliden oder $C_{2-4}$-Alkenyl ist, wobei die Summe der C-Atome in diesen Resten 2-5 beträgt, m und n = 0 oder 1 sind, p = 1, 2 oder 3 ist, und die gestrichelten Linien eine oder zwei fakultative Doppelbindungen darstellen, dadurch gekennzeichnet, dass man ein Enamin von Cyclopentanon mit einem Säurehalogenid der Formel

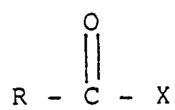

(II)

worin R obige Bedeutung hat und X Halogen darstellt, umsetzt, wobei die Herstellung von 2-[3,3,5-Trimethyl-cyclohexylacetyl]-cyclopentanon ausgeschlossen ist.

13. Verwendung von Verbindungen der Formel

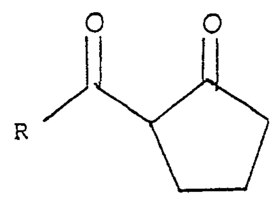

(I)

worin R einen aliphatischen Rest der Formel

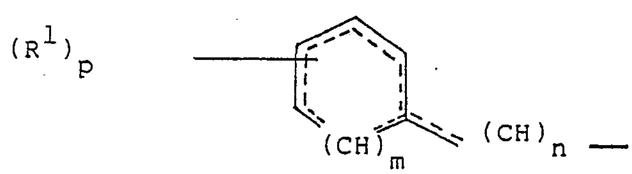

bedeutet, worin $R^1$ $C_{1-4}$-Alkyl, $C_{1-4}$-Alkyliden oder $C_{2-4}$-Alkenyl ist, wobei die Summe der C-Atome in diesen Resten 2-5 beträgt, m und n = 0 oder 1 sind, p = 1, 2 oder 3 ist, und die gestrichelten Linien eine oder zwei fakultative Doppelbindungen darstellen, als Riechstoffe.

14. Verwendung von 2-[3,3,5-Trimethyl-cyclohexyl-acetyl]-cyclopentanon als Riechstoff.

15. Verwendung von 2-[3,3,5-Trimethyl-1- oder 6-cyclohexenyl bzw. cyclohexyliden-acetyl] cyclopentanon als Riechstoff.

16. Verwendung von 2-[1-Methyl-2-methylen-3-(prop-1-en-2-yl)-cyclopentylcarbonyl]-cyclopentanon als Riechstoff.

**Claims**

1. Compounds of the formula

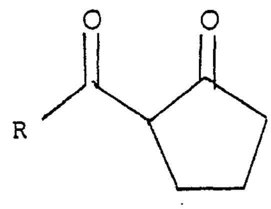

(I)

wherein R signifies an aliphatic residue of the formula

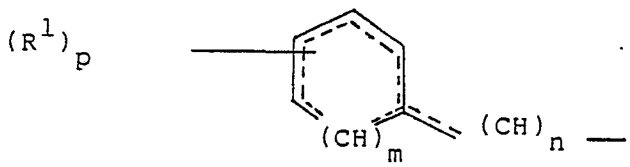

in which $R^1$ is $C_{1-4}$-alkyl, $C_{1-4}$-alkylidene or $C_{2-4}$-alkenyl, whereby the sum of the C-atoms in these residues amounts to 2-5, m and n are = 0 or 1, p is = 1, 2 or 3, and the dotted lines respresent one or two optional double bonds, with the exception of 2-[3,3,5-trimethyl-cyclohexylacetyl]-cyclopentanone.

2. 2-[3,3,5-Trimethyl-1- or 6-cyclohexenyl or cyclohexylidene-acetyl]-cyclopentanone.

3. 2-[1-Methyl-2-methylene-3-(prop-1-en-2-yl)-cyclopentylcarbonyl]-cyclopentanone.

4. 2-[3,5-Dimethyl-cyclohexylacetyl]-cyclopentanone.

5. 2-[3,4-Dimethyl-cyclohexylacetyl]-cyclopentanone.

6. 2-[1,2-Dimethyl-3-isopropyl-cyclopentylcarbonyl]-cyclopentanone.

7. 2-[2-Sec.butyl-cyclohexylactyl]-cyclopentanone.

8. Odorant substance compositions characterized by a content of a compound of the formula

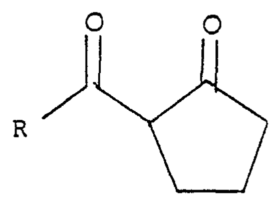

(I)

wherein R signifies an aliphatic residue of the formula

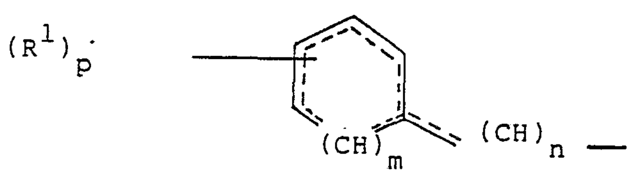

in which $R^1$ is $C_{1-4}$-alkyl, $C_{1-4}$-alkylidene or $C_{2-4}$-alkenyl, whereby the sum of the C-atoms in these residues amounts to 2-5, m and n are = 0 or 1, p is = 1, 2 or 3, and the dotted lines respresent one or two optional double bonds.

9. An odorant substance composition, characterized by a content of 2-[3,3,5-trimethyl-cyclohexyl-acetyl]-cyclopentanone.

10. An odorant substance composition, characterized by a content of 2-[3,3,5-trimethyl-1- or 6-cyclohexenyl or cyclohexylidene-acetyl]-cyclopentanone.

11. An odorant substance composition, characterized by a content of 2-[1-methyl-2-methylene-3-(prop-1-en-2-yl)-cyclopentylcarbonyl]-cyclopentanone.

12. A process for the manufacture of compounds of the formula

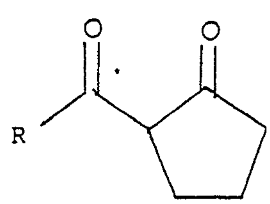

(I)

wherein R signifies an aliphatic residue of the formula

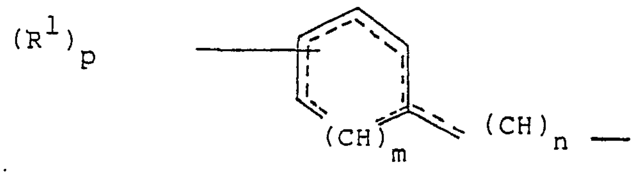

in which $R^1$ is $C_{1-4}$-alkyl, $C_{1-4}$-alkylidene or $C_{2-4}$-alkenyl, whereby the sum of the C-atoms in these residues amounts to 2-5, m and n are = 0 or 1, p is = 1, 2 or 3, and the dotted lines respresent one or two optional double bonds, characterized by reacting an enamine of cyclopentanone with an acid halide of the formula

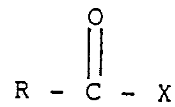

(II)

wherein R has the above significance and X represents halogen, whereby the manufacture of 2-[3,3,5-trimethyl-cyclohexyl-acetyl]-cyclopentanone is excluded.

13. The use of compounds of the formula

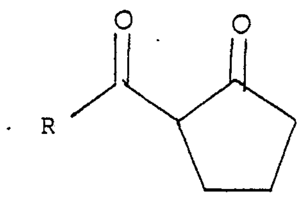

(I)

wherein R signifies an aliphatic residue of the formula

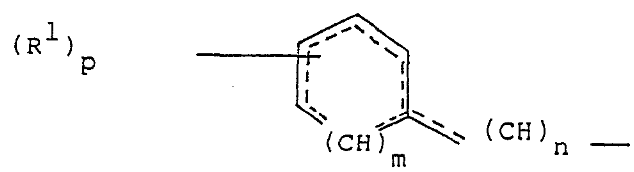

in which $R^1$ is $C_{1-4}$-alkyl, $C_{1-4}$-alkylidene or $C_{2-4}$-alkenyl, whereby the sum of the C-atoms in these residues amounts to 2-5, m and n are = 0 or 1, p is = 1, 2 or 3, and the dotted lines respresent one or two optional double bonds, as odorant substances.

14. The use of 2-[3,3,5-trimethyl-cyclohexylacetyl]-cyclopentanone as an odorant substance.

15. The use of 2-[3,3,5-trimethyl-1- or 6-cyclohexenyl or cyclohexylidene-acetyl]-cyclopentanone as an odorant substance.

16. The use of 2-[1-methyl-2-methylene-3-(prop-1-en-2-yl)-cyclopentylcarbonyl]-cyclopentanone as an odorant substance.

**Revendications**

1. Composés de formule

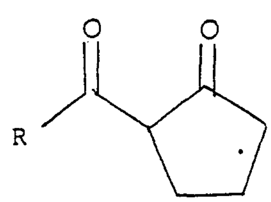

(I)

dans laquelle R représente un reste aliphatique de formule

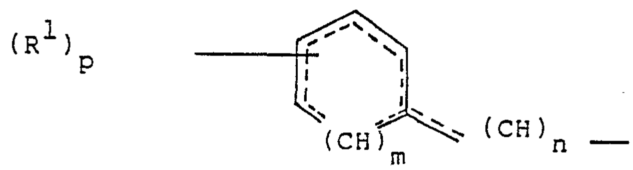

dans laquelle $R^1$ représente un reste alkyle en $C_1$-$C_4$, alkylidène en $C_1$-$C_4$ ou alcényle en $C_2$-$C_4$, la somme des atomes de carbone de ces restes allant de 2 à 5, m et n = 0 ou 1, p = 1, 2 ou 3, et les traits interrompus représentent une ou deux doubles liaisons facultatives, à l'exception de la 2-(3,3,5-triméthyl-cyclohexylacétyl)-cyclopentanone.

2. La 2-(3,3,5-triméthyl-1- ou 6-cyclohexényl ou cyclohexylidène-acétyl)-cyclopentanone.

3. La 2-[1-méthyl-2-méthylène-3-(propa-1-ène-2-yl)-cyclopentylcarbonyl]-cyclopentanone.

4. La 2-(3,5-diméthyl-cyclohexylacétyl)-cyclopentanone.

5. La 2-(3,4-diméthyl-cyclohexylacétyl)-cyclopentanone.

6. La 2-(1,2-diméthyl-3-isopropyl-cyclopentylcarbonyl)-cyclopentanone.

7. La 2-(2-sec.-butyl-cyclohexylacétyl)-cyclopentanone.

8. Compositions de parfums caractérisées en ce qu'elles contiennent un composé de formule

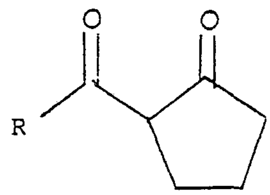

(I)

dans laquelle R représente un reste aliphatique de formule.

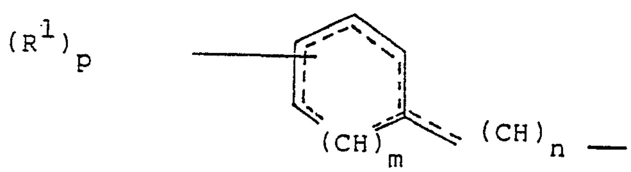

dans laquelle $R^1$ représente un reste alkyle en $C_1$-$C_4$, alkylidène en $C_1$-$C_4$ ou alcényle en $C_2$-$C_4$, la somme des atomes de carbone de ces restes allant de 2 à 5, m et n = 0 ou 1, p = 1, 2 ou 3, et les traits interrompus représentent une ou deux doubles liaisons facultatives.

9. Composition de parfum caractérisée en ce qu'elle contient de la 2-(3,3,5-triméthyl-cyclohexylacétyl)-cyclopentanone.

10. Composition de parfum caractérisée en ce qu'elle contient de la 2-(3,3,5-triméthyl-1- ou 6-cyclohexényl ou cyclohexylidène-acétyl)-cyclopentanone.

11. Composition de parfum caractérisée en ce qu'elle contient de la 2-[1-méthyl-2-méthylène-3-(propa-1-ène-2-yl)-cyclopentylcarbonyl]-cyclopentanone.

12. Procédé de préparation des composés de formule

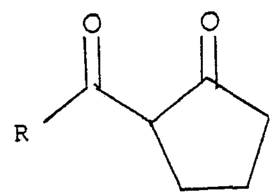

(I)

dans laquelle R représente un reste aliphatique de formule

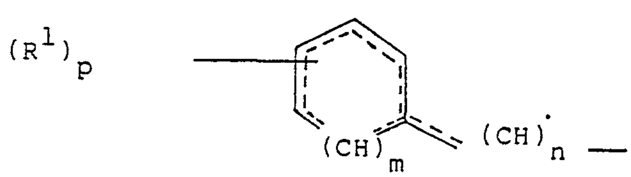

dans laquelle $R^1$ représente un reste alkyle en $C_1$-$C_4$, alkylidène en $C_1$-$C_4$ ou alcényle en $C_2$-$C_4$, la

somme des atomes de carbone de ces restes allant de 2 à 5, m et n = 0 ou 1, p = 1, 2 ou 3, et les traits interrompus représentent une ou deux doubles liaisons facultatives, caractérisé en ce que l'on fait réagir une énamine de cyclopentanone avec un halogénure d'acide de formule

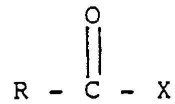

(II)

dans laquelle R a les significations indiquées ci-dessus et X représente un halogène, à l'exclusion de la préparation de la 2-(3,3,5-triméthyl-cyclohexylacétyl)-cyclopentanone.

13. Utilisation de composés de formule

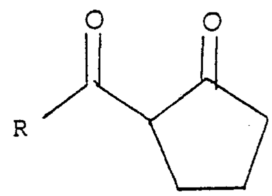

(I)

dans laquelle R représente un reste aliphatique de formule

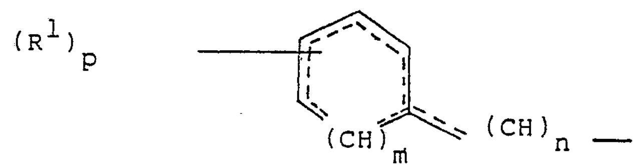

dans laquelle $R^1$ représente un reste alkyle en $C_1$-$C_4$, alkylidène en $C_1$-$C_4$ ou alcényle en $C_2$-$C_4$, la somme des atomes de carbone de ces restes allant de 2 à 5, m et n = 0 ou 1, p = 1, 2 ou 3, et les traits interrompus représentent une ou deux doubles liaisons facultatives, en tant que matières odorantes.

14. Utilisation de la 2-(3,3,5-triméthyl-cyclohexylacétyl)-cyclopentanone en tant que matière odorante.

15. Utilisation de la 2-(3,3,5-triméthyl-1- ou 6-cyclohexényl ou cyclohexylidène-acétyl)-cyclopentanone en tant que matière odorante.

16. Utilisation de la 2-[1-méthyl-2-méthylène-3-(propa-1-ène-2yl)-cyclopentylcarbonyl]-cyclopentanone en tant que matière odorante.